# EUROPÄISCHE PATENTSCHRIFT

(11) **EP 3 254 714 B1**
(45) Veröffentlichungstag und Bekanntmachung des Hinweises auf die Patenterteilung: **28.07.2021**
(21) Anmeldenummer: 16173551.9
(22) Anmeldetag: 08.06.2016
(51) Int. Cl.: A61M 39/00, A61M 5/142, A61M 1/00, A61M 5/145

(54) **VORRICHTUNG UND SYSTEM ZUR ABGABE EINES FLUIDS UNTER ASEPTISCHEN BEDINGUNGEN**
DEVICE AND SYSTEM FOR DISPENSING A FLUID UNDER ASEPTIC CONDITIONS
DISPOSITIF ET SYSTEME DE DISTRIBUTION D'UN FLUIDE DANS DES CONDITIONS ASEPTIQUES

(43) Veröffentlichungstag der Anmeldung: 13.12.2017
(73) Patentinhaber: SHL Medical AG, 6302 Zug (CH)
(72) Erfinder: WEIBEL, Ludwig Daniel, 9104 Waldstatt (CH); WYLER, Samuel, 9030 Abtwil (CH)
(74) Vertreter: SHL Medical

(56) Entgegenhaltungen:
- WO-A1-2014/015257
- WO-A1-2014/191038
- WO-A2-2008/024814
- US-A1- 2010 174 266
- US-A1- 2016 082 201

## Beschreibung

Die vorliegende Erfindung betrifft eine Vorrichtung und ein System zur Abgabe eines Fluids unter aseptischen Bedingungen, insbesondere unter aseptischen Bedingungen, gemäss den Oberbegriffen des unabhängigen Anspruchs.

Eine aus dem Stand der Technik bekannte Vorrichtung dieser Art zeigt Dokument WO 2014/0157257.

Zur parenteralen Injektion flüssiger Formulierungen pharmazeutischer Wirkstoffe haben sich neben Injektionsspritzen und Arzneimittelstiften (sog. Pens) auch Arzneimittelpumpen in der jüngeren Vergangenheit bewährt. Derartige Pumpen sind insbesondere vorteilhaft, wenn ein Präparat nach einem speziell vorgegebenen Schema, beispielsweise über einen längeren Zeitraum, unabhängig von der Anwesenheit von medizinischem Personal an einem Patienten abgegeben werden muss. Die Pumpen können je nach Anwendung am Körper des Patienten angebracht werden und umfassen typischerweise einen Behälter für die flüssige Formulierung sowie eine Fördervorrichtung, die diese zu einem Anschluss oder zu einem Injektionssystem fördert. Das Injektionssystem kann eine Verweilkanüle umfassen, die während des gesamten Verabreichungszeitraumes im Körper des Patienten verbleibt.

Das Arzneimittel Neulasta® enthält den Wirkstoff Pegfilgrastim, der zu der Gruppe der Zytokine gehört und einem körpereigenem Protein, dem Granulozyten-Kolonien-stimulierenden-Faktor, sehr ähnlich ist. Neulasta® wird zur Verkürzung der Dauer von Neutropenien (niedrige Anzahl der weissen Blutkörperchen) und zur Verminderung des Vorkommens neutropensischen Fibers (niedrige Anzahl der weissen Blutkörperchen in Verbindung mit Fieber) eingesetzt. Diese Zustände können durch den Einsatz einer zytotoxischen Chemotherapie (Arzneimittel, die schnellwachsende Zellen zerstören) hervorgerufen werden. Wenn die Anzahl der weissen Blutkörperchen auf einen niedrigen Spiegel abfällt, stehen dem Körper möglicherweise nicht mehr genügend dieser Zellen zur Bekämpfung von Krankheitserregern zur Verfügung, was zu einem erhöhten Infektionsrisiko führt.

Neulasta® ist als Fertigspritze erhältlich, die eine einzelne Dosis von 6 mg in 0.6 ml Injektionslösung enthält. Die Lösung wird subkutan (d.h. unter die Haut) gespritzt. Die Gabe muss dabei nach einem streng vorgegebenen Zeitschema erfolgen, üblicherweise 24h nach der letzten Dosis des Chemotherapeutikums am Ende eines jeden Behandlungszyklus. Erfolgt die Verabreichung nicht gemäss den Vorgaben, kann das Präparat unter Umständen wirkungslos sein.

Um die Verabreichung von Neulasta® für den Patienten zu vereinfachen, wird das in einer Einwegspritze gelieferte Medikament als Set mit einer Arzneimittelpumpe (Markenname: Onpro™) angeboten. Die besagte Arzneimittelpumpe muss von medizinischem Fachpersonal über eine Einfüllöffnung mit der Lösung aus der Spritze befüllt werden. Einmal am Körper des Patienten angebracht, erfolgt 27 Stunden nach Aktivierung der Pumpe die Abgabe des Medikamentes über einen Zeitraum von 45 Minuten. Die Verwendung der besagten Arzneimittelpumpe hat den Vorteil, dass ein erneuter Arztbesuch, einzig zur Verabreichung von Neulasta®, nicht mehr erforderlich ist. Da sich der Patient das Präparat auch nicht selbst injizieren muss, wird verhindert, dass dieser die Verabreichung vergisst oder ihm dabei Fehler unterlaufen (gerade im Hinblick auf seinen Gesundheitszustand).

Die besagte Arzneimittelpumpe Onpro™ weist allerdings verhältnismässig viele Einzelteile auf, weshalb sie aufwendig und kostenintensiv in der Fertigung ist. Zudem muss die Lösung mit der vorgefüllten Spritze über ein vergleichsweise kleines Septum in die Arzneimittelpumpe eingefüllt werden. Dies kann nur von medizinischem Personal bewerkstelligt werden. Darüber hinaus besteht die Gefahr, dass es beim Umfüllen der Lösung zu einer Kontamination derselben kommt.

Es ist daher eine Aufgabe der vorliegenden Erfindung, die Nachteile im Stand der Technik zu überwinden. Insbesondere ist es eine Aufgabe der Erfindung, eine vielfältig anwendbare und konstruktiv einfache Vorrichtung zur Abgabe eines Fluids unter aseptischen Bedingungen zu schaffen, die preisgünstiger herzustellen ist. Zudem soll die Vorrichtung für die Handhabung durch einen Patienten geeignet sein.

Diese Aufgaben werden durch eine Vorrichtung sowie ein System gelöst, welche die Merkmale in Anspruch 1 aufweisen.

Die Vorrichtung zur Abgabe eines Fluids unter aseptischen Bedingungen umfasst eine Fördervorrichtung zur Förderung des Fluids zu einer Abgabeöffnung. Ferner umfasst die Vorrichtung ein Kopplungselement zum Ankoppeln einer Spritze. Das Fluid ist von der Spritze über das Kopplungselement und die Fördervorrichtung ausschliesslich unter Einwirkung der Fördervorrichtung zur Abgabeöffnung förderbar.

Insbesondere erzeugt die Fördervorrichtung dabei auf der Seite der Spritze einen Unterdruck, wodurch der Inhalt der Spritze aus dieser ausgesaugt wird und sich der Kolben der Spritze durch die Saugkraft in Richtung des proximalen Endes der Spritze bewegt. Es versteht sich von selbst, dass die Fördervorrichtung hierzu eine ausreichende Saugkraft bereitstellen muss.

Durch diese Ausgestaltung der Vorrichtung kann auf einen separaten Behälter zur Aufnahme des Fluids verzichtet werden. Diese Funktion wird von der Spritze übernommen, die in vielen Fällen von Seiten des Herstellers mit dem Fluid vorgefüllt ist. Dadurch kann die Gesamtzahl der Teile des Systems reduziert werden, was in einer höheren Zuverlässigkeit und in geringeren Kosten resultiert. Da Pharmazeutika strengen Regulatorien unterliegen und die Einführung einer neuen Verpackung registrierungspflichtig ist, ist es ferner vorteilhaft, dass eine klassische Spritze sowohl zur Injektion als auch in Verbindung mit der Vorrichtung verwendet werden kann. Damit können aufwendige und kostenintensive weitere Zulassungsverfahren vermieden werden. Da das Fluid nicht noch in einen weiteren Behälter transferiert werden muss, sondern direkt von der Spritze über die Fördervorrichtung zum Patienten transferiert wird, kann zudem das Risiko einer Kontamination des Fluids und damit einer Gefährdung des Patienten verringert werden.

Das Kopplungselement umfasst ein von einer Spritzennadel oder Kanüle penetrierbares Septum. Dies ist insbesondere vorteilhaft, wenn die Spritze eine fest angebrachte Spritzennadel oder Kanüle umfasst, wie es bei Fertigspritzen im Allgemeinen üblich ist. Allerdings ist diese Ausführung auch bei Spritzen mit auswechselbarer Nadel vorteilhaft, da die Verbindung zwischen der Spritze und der Fördervorrichtung so auf eine überaus hygienische Weise hergestellt werden kann.

Das Kopplungselement ist als Nadelaufnahme ausgebildet, in welche das Septum eingesetzt ist, insbesondere in eine umlaufende Nut. Die Nadelaufnahme kann als, insbesondere gekrümmter, Leitungsabschnitt ausgebildet sein. Die Ausbildung des Kopplungselementes als Nadelaufnahme hat den Vorteil, dass die Nadel für das Durchstechen des Septums kontrolliert geführt und danach sicher gehalten werden kann. Die Nadelaufnahme kann einstückig mit dem Gehäuse der Vorrichtung ausgebildet sein. Dies ermöglicht es, eine derartige Vorrichtung mit Nadelaufnahme konstruktiv einfach zu halten und auf effiziente, kostengünstige Weise zu fertigen.

Die Nadelaufnahme kann mit einem Leitungselement, insbesondere einer Stahlkanüle, in Fluidkommunikation stehen, wobei das Leitungselement bevorzugt in eine Wand der Nadelaufnahme eingebettet, insbesondere umspritzt ist oder über ein Dichtungselement, insbesondere ein weiteres Septum, welches vorzugsweise in eine umlaufende Nut der Nadelaufnahme eingesetzt ist, mit der Nadelaufnahme verbunden sein. Über das Leitungselement kann die Nadelaufnahme mit weiteren Teilen der Vorrichtung, insbesondere mit der Fördervorrichtung fluidmässig verbunden sein. Das Einbetten des Leitungselementes in eine Wand der Nadelaufnahme oder die Verwendung eines Dichtungselementes stellen dabei konstruktiv vorteilhafte Varianten dar, um das Leitungselement mit der Nadelaufnahme zu verbinden.

Die Vorrichtung umfasst eine Halterung, in welche die Spritze einlegbar ist, wobei die Spritze, wenn sie in die Halterung eingelegt ist, über das Kopplungselement an die Vorrichtung ankoppelbar ist. Eine Halterung hat den Vorteil, dass die Spritze, wenn sie mit der Vorrichtung verbunden ist, sicher an ihrer vorgesehenen Position gehalten wird. Darüber hinaus kann durch die Halterung das Ankoppeln der Spritze an das Kopplungselement vereinfacht, insbesondere geführt werden. Dadurch kann eine derartige Vorrichtung auch von nicht medizinisch geschultem Personal, insbesondere vom Patienten selbst, sicher verwendet werden.

Die Halterung ist als Einschub ausgebildet, der in einen Einschubbereich der Vorrichtung einschiebbar ist. Dadurch, dass die Spritze erst in einen Einschub eingelegt wird, bevor dieser in die Vorrichtung eingeschoben wird und damit ein Ankoppeln der Vorrichtung an das Kopplungselement erfolgt, kann die Verwendung der Vorrichtung weiter vereinfacht werden. Der Einschub kann ein Abziehelement aufweisen, mit dem eine Schutzkappe einer in den Einschub eingelegten Spritze abziehbar ist. Dies kann die Verwendung der Vorrichtung, insbesondere für einen Patienten, weiter vereinfachen.

Ein weiterer Aspekt der vorliegenden Erfindung betrifft ein System zur Abgabe eines Fluids unter aseptischen Bedingungen umfassend eine Vorrichtung wie vorgängig beschrieben und eine mit dem Kopplungselement koppelbare Spritze. Durch das Bereitstellen von Vorrichtung und Spritze als System kann mit der Vorrichtung eine Spritze geliefert werden, die speziell auf diese abgestimmt ist. Dies bezieht sich insbesondere auf die Dimensionen der Spritze, beispielsweise auf die Länge und den Durchmesser einer fest angebrachten Spritzennadel und auf deren allgemeine Konstruktionsweise. So ist es beispielsweise möglich, dass die Spritze ohne Kolbenstange ausgeführt ist. Dies verringert insbesondere die Gesamtlänge der Spritze bei zurückgezogenem Kolben, was vor allem bei vorgefüllten Fertigspritzen vorteilhaft ist, um die Grösse der Vorrichtung und der Verpackung der Spritze zu reduzieren.

Die Spritze kann eine insbesondere fest am Spritzenkörper angebrachte Hohlnadel oder Kanüle aufweisen. Dies ist heutzutage bei Fertigspritzen gängig. Einerseits kann damit eine Kontamination des Fluids beim Anbringen einer separaten Spritzennadel vermieden werden. Andererseits führt dies zu einer Vereinfachung der Verwendung, da ein separater Schritt für das Anbringen einer Spritzennadel oder Kanüle entfällt.

Die Vorrichtung umfasst einen Spritzenhalter, welcher derart ausgestaltet ist, dass die Spritzennadel oder Kanüle einer darin eingelegten Spritze abgeschirmt ist. Damit kann im Wesentlichen verhindert werden, dass sich ein Anwender der Vorrichtung nach Entfernen der Schutzkappe an der Spritzennadel verletzt.

Weitere Vorteile und Einzelmerkmale der Erfindung ergeben sich aus der nachfolgenden Beschreibung eines Ausführungsbeispiels und aus den Zeichnungen.

Es zeigen schematisch:
- Figur 1:: Perspektivische Darstellung der Oberseite einer erfindungsgemässen Vorrichtung;
- Figur 2:: Perspektivische Darstellung der Unterseite der Vorrichtung gemäss Figur 1;
- Figur 3:: Perspektivische Darstellung der Vorrichtung gemäss den Figuren 1 und 2, jedoch mit separat dargestellter Halterung, welche als Einschub ausgebildet ist, in welchen eine Spritze eingelegt ist;
- Figuren 4 bis 7:: Perspektivische Darstellung einer Folge von Schritten, welche das Einlegen einer Spritze in den Einschub einer Vorrichtung gemäss den Figuren 1 bis 3 sowie das Abziehen der Schutzkappe der Spritze darstellt.

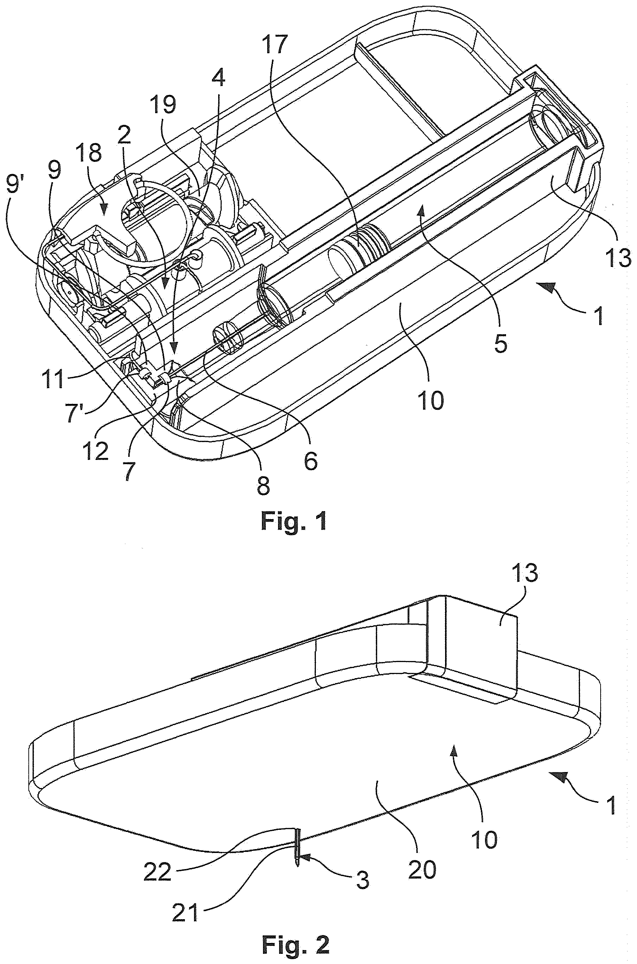

Figur 1 zeigt eine erfindungsgemässe Vorrichtung 1, wobei zur besseren Übersichtlichkeit der Oberteil eines Gehäuses 10 weggelassen ist. Zudem sind einzelne Teile der Vorrichtung 1 wie beispielsweise ein Förderantrieb oder eine Steuerungselektronik nicht dargestellt. Bei der dargestellten Vorrichtung ist eine Halterung für die Spritze 5 als Einschub 13 ausgestaltet, welcher hier in die Vorrichtung 1 eingeschoben ist. Die Spritze 5 weist einen Kolben 17 auf, an dem im Gegensatz zu gängigen Spritzen keine Kolbenstange angebracht ist. Zudem weist die Spritze 5 eine fest angebrachte Spritzennadel 6 auf. Es ist zu erkennen, dass die Spritzennadel 6 in ein Kopplungselement 4 hineinragt, welches im vorliegenden Beispiel als Nadelaufnahme 8 ausgebildet ist. Der der Spritze 5 zugewandte Bereich der Nadelaufnahme 8 ist dabei trichterförmig ausgeführt um die Spritzennadel 6 in Richtung eines Septums 7 zu leiten. Das Septum 7 ist in einer umlaufenden Nut 9 einer Wand 12 der Nadelaufnahme 8 eingebettet. Die Nadelaufnahme 8 ist als gekrümmter Leitungsabschnitt ausgebildet, welcher einstückig mit dem Gehäuse 10 der Vorrichtung 1 ausgebildet ist. Die Nadelaufnahme 8 ist über ein Leitungselement 11, welches hier als Stahlkanüle ausgebildet ist, mit der Fördervorrichtung 2 verbunden. Der fluidmässige Anschluss des Leitungselementes 11 an die Nadelaufnahme 8 erfolgt über ein Septum 7', welches ebenfalls in eine umlaufende Nut 9' innerhalb der Wand 12 der Nadelaufnahme 8 eingebettet ist. Neben der Fördervorrichtung 2 verfügt die Vorrichtung 1 über eine Injektionsvorrichtung 18, welche beide über eine von einem nicht dargestellten Förderantrieb angetriebene Kurventrommel 19 angetrieben bzw. gesteuert werden.

Aus Figur 2 ist eine Unterseite der Vorrichtung 1 ersichtlich. Es ist zu erkennen, dass das Gehäuse 10 dort eine Kontaktfläche 20 aufweist, über die die Vorrichtung 1, insbesondere über einen Klebefilm, an den Körper eines Patienten anbringbar ist. Im gezeigten Ausführungsbeispiel der Vorrichtung 1 ist eine Abgabeöffnung 3 als eine Kombination von Einstechkanüle und Verweilkanüle 21 ausgeführt, welche über einen Setzmechanismus der Injektionsvorrichtung 18 durch die Öffnung 22 im Gehäuse 10 gesetzt werden.

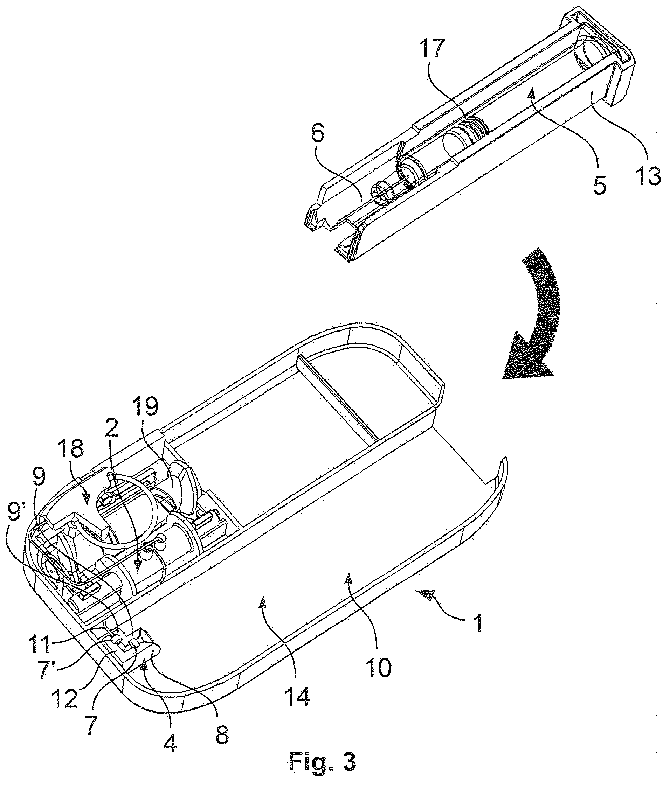

In Figur 3 sind die vorgängig beschriebene Vorrichtung 1 und der Einschub 13 mit der Spritze 5 separat dargestellt. Es ist zu erkennen, dass die Spritzennadel 6 durch den Einschub 5 weitestgehend abgeschirmt ist, wodurch verhindert werden kann, dass sich ein Benutzer damit verletzt. Der Einschub 13 wird in einen Einschubbereich 14 der Vorrichtung 1 eingeschoben, wodurch die Spritzennadel 6 in die Nadelaufnahme 8 eingeführt und das Septum 7 durchstochen wird.

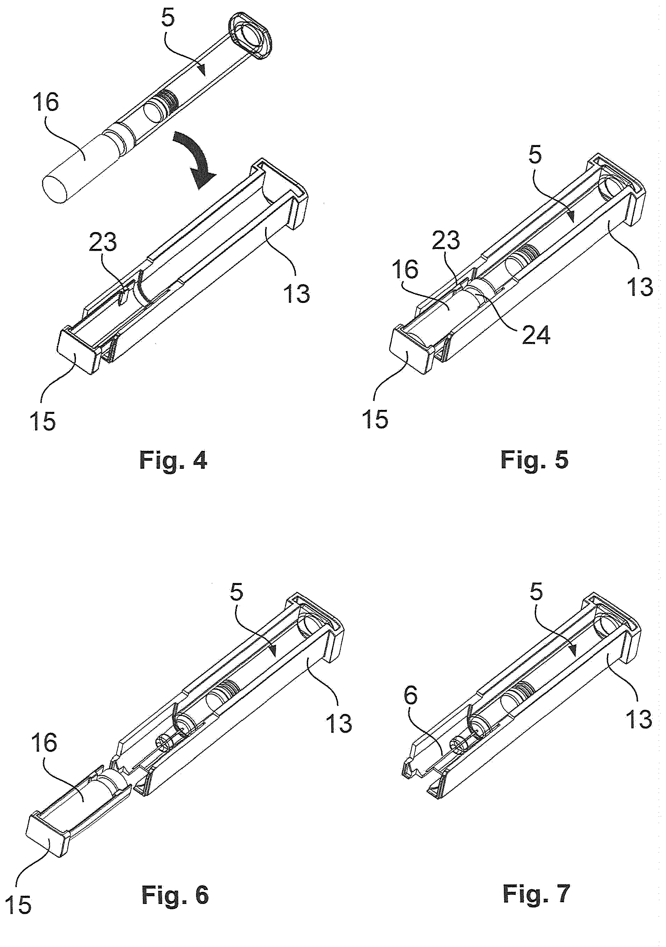

Aus den Figuren 4 bis 7 ist das Einlegen der Spritze 5 in den Einschub 13 und das Abziehen einer Schutzkappe 16 von der Spritze 5 dargestellt. In Figur 4 sind der Einschub 13 mit einem Abziehelement 15 einerseits und die Spritze 5 mit der Schutzkappe 16 andererseits einzeln dargestellt. In Figur 5 ist die Spritze 5 in den Einschub 13 eingelegt. Es ist zu erkennen, dass Eingriffelemente 23 des Abziehelementes 15 in eine Nut 24 der Schutzkappe 16 eingreifen. In Figur 6 ist die Schutzkappe 16 mit dem Abziehelement 15 bereits von der Spritze 5 abgezogen. Figur 7 zeigt das Abziehelement 13 mit der eingelegten Spritze 5 bereit zum Einschieben in die Vorrichtung 1. Es ist zu erkennen, dass die Spritzennadel 6 der Spritze 5 durch den Einschub 13 im Wesentlichen abgeschirmt wird, so dass verhindert werden kann, dass sich ein Benutzer an der Spritzennadel 6 verletzt.

## Patentansprüche

1. Vorrichtung (1) zur Abgabe eines Fluids unter aseptischen Bedingungen, umfassend eine Fördervorrichtung (2) zur Förderung des Fluids zu einer Abgabeöffnung (3), und weiter umfassend ein Kopplungselement (4) zum Ankoppeln einer Spritze (5)eine Spritzennadel (6) oder Kanüle umfassend, wobei das Kopplungselement (4) ein von einer Spritzennadel oder Kanüle (6) penetrierbares Septum (7) umfasst, wobei das Kopplungselement (4) als Nadelaufnahme (8) ausgebildet ist, in welche das Septum (7) eingesetzt ist, insbesondere in eine umlaufende Nut (9), wobei das Fluid von der Spritze (5) über das Kopplungselement (4) und die Fördervorrichtung (2) ausschliesslich unter Einwirkung der Fördervorrichtung (2) zur Abgabeöffnung (3) förderbar ist, wobei die Vorrichtung eine Halterung (13) umfasst, in welche die Spritze (5) einlegbar ist, wobei die Spritze (5), wenn sie in die Halterung (13) eingelegt ist, über das Kopplungselement (4) an die Vorrichtung (1) ankoppelbar ist;
wobei die Halterung als Einschub (13) ausgebildet ist, der in einen Einschubbereich (14) der Vorrichtung (1) einschiebbar ist, wobei die Spritzennadel (6) durch den Einschub (5) weitestgehend abgeschirmt wird.

2. Vorrichtung (1) nach Anspruch 1, wobei die Nadelaufnahme (8) als, insbesondere gekrümmter, Leitungsabschnitt ausgebildet ist.

3. Vorrichtung (1) nach einem der Ansprüche 1 oder 2, wobei die Nadelaufnahme (8) einstückig mit einem Gehäuse (10) der Vorrichtung (1) ausgebildet ist.

4. Vorrichtung (1) nach einem der Ansprüche 1 bis 3, wobei die Nadelaufnahme (8) mit einem Leitungselement (11), insbesondere einer Stahlkanüle, in Fluidkommunikation steht, wobei das Leitungselement (11) bevorzugt in eine Wand (12) der Nadelaufnahme (8) eingebettet, insbesondre umspritzt, ist oder über ein Dichtungselement (7'), insbesondere ein weiteres Septum, welches vorzugsweise in eine umlaufende Nut (9') der Nadelaufnahme (8) eingesetzt ist, mit der Nadelaufnahme (8) verbunden ist.

5. Vorrichtung (1) nach Anspruch 4, wobei der Einschub (13) ein Abziehelement (15) aufweist, mit dem eine Schutzkappe (16) einer in den Einschub (13) eingelegten Spritze (5) abziehbar ist.

6. System zur Abgabe eines Fluids unter aseptischen Bedingungen umfassend eine Vorrichtung (1) nach einem der Ansprüche 1 bis 8 und eine mit dem Kopplungselement (4) koppelbare Spritze (5).

7. System nach Anspruch 6, wobei die Spritze (5) ohne Kolbenstange ausgeführt ist.

8. System nach einem der Ansprüche 6 oder 7, wobei die Spritze (6) eine insbesondere fest am Spritzenkörper angebrachte Hohlnadel oder Kanüle (6) aufweist.

9. System nach Anspruch 8, wobei die Vorrichtung (1) einen Spritzenhalter (13) umfasst, welcher derart ausgestaltet ist, dass die Spritzennadel oder Kanüle (6) einer darin eingelegten Spritze (5) abgeschirmt ist.

## Claims

1. Device (1) for dispensing a fluid under aseptic conditions, comprising a conveying device (2) for conveying the fluid to a dispensing opening (3), and further comprising a coupling element (4) for connecting a syringe (5)comprising a syringe needle (6) or cannula, wherein the coupling element (4) comprises a septum (7) that can be penetrated by a syringe needle or cannula (6); wherein the coupling element (4) is designed as a needle receptacle (8) into which the septum (7) is inserted, in particular into a circumferential groove (9); wherein the fluid can be conveyed from the syringe (5), via the coupling element (4) and the conveying device (2), to the dispensing opening (3) exclusively under the action of the conveying device (2); wherein the device comprises a holder (13) into which the syringe (5) can be inserted; wherein the syringe (5) - when inserted into the holder (13) - can be coupled to the device (1) via the coupling element (4); wherein the holder is designed as a plug-in unit (13) which can be inserted into a plug-in region (14) of the device (1); wherein the syringe needle (6) is largely shielded by the plug-in unit (5).

2. Device (1) according to claim 1, wherein the needle receptacle (8) is designed as a conduit segment, in particular a curved conduit segment.

3. Device (1) according to any one of claims 1 or 2, wherein the needle receptacle (8) is formed integrally with a housing (10) of the device (1).

4. Device (1) according to any one of claims 1 to 3, wherein the needle receptacle (8) is in fluid communication with a conduit element (11), in particular a steel cannula; wherein the conduit element (11) is preferably embedded, especially is extrusion-coated, in a wall (12) of the needle receptacle (8), or is connected to the needle receptacle (8) via a sealing element (7'), in particular a further septum, which is preferably inserted into a circumferential groove (9') of the needle receptacle (8).

5. Device (1) according to claim 4, wherein the plug-in unit (13) has a removal element (15) by means of which a protective cap (16) of a syringe (5) inserted into the plug-in unit (13) can be pulled off.

6. System for dispensing a fluid under aseptic conditions, comprising a device (1) according to any one of claims 1 to 8 and a syringe (5) which can be coupled to the coupling element (4).

7. System according to claim 6, wherein the syringe (5) is designed without a plunger rod.

8. System according to any one of claims 6 or 7, wherein the syringe (6) has a hollow needle or cannula (6) which is in particular fixedly attached to the syringe body.

9. System according to claim 8, wherein the device (1) comprises a syringe holder (13) which is designed such that the syringe needle or cannula (6) of a syringe (5) inserted therein is shielded.

## Revendications

1. Dispositif (1) de distribution d'un fluide dans des conditions aseptiques, comprenant un dispositif de transport (2) destiné au transport du fluide jusqu'à une ouverture de distribution (3) et comprenant en outre un élément d'accouplement (4) destiné à l'accouplement d'une seringue (5) comprenant une aiguille de seringue (6) ou canule, dans lequel l'élément d'accouplement (4) comprend un septum (7) pouvant être pénétré par une aiguille de seringue ou canule (6), dans lequel l'élément d'accouplement (4) est conçu comme un réceptacle d'aiguille (8), dans lequel le septum (7) est introduit, en particulier dans une rainure circulaire (9), dans lequel le fluide peut être transporté depuis la seringue (5) par l'intermédiaire de l'élément d'accouplement (4) et du dispositif de transport (2) uniquement sous l'action du dispositif de transport (2) jusqu'à l'ouverture de distribution (3), dans lequel le dispositif comprend un support (13), dans lequel la seringue (5) peut être placée, dans lequel la seringue (5), lorsqu'elle est placée dans le support (13), peut être accouplée au dispositif (1) par l'intermédiaire de l'élément d'accouplement (4) ; dans lequel le support est conçu comme un tiroir (13), qui peut être inséré dans une zone d'insertion (14) du dispositif (1), dans lequel l'aiguille de seringue (6) est protégée autant que possible par le tiroir (5).

2. Dispositif (1) selon la revendication 1, dans lequel le réceptacle d'aiguille (8) est conçu comme une section de ligne, en particulier incurvée.

3. Dispositif (1) selon l'une quelconque des revendications 1 ou 2, dans lequel le réceptacle d'aiguille (8) est conçu d'une seule pièce avec un boîtier (10) du dispositif (1).

4. Dispositif (1) selon l'une quelconque des revendications 1 à 3, dans lequel le réceptacle d'aiguille (8) se trouve en communication fluidique avec un élément de ligne (11), en particulier une canule d'acier, dans lequel l'élément de ligne (11) est de préférence intégré, en particulier surmoulé, dans une paroi (12) du réceptacle d'aiguille (8) ou est relié par l'intermédiaire d'un élément d'étanchéité (7'), en particulier un autre septum, qui est de préférence introduit dans une rainure circulaire (9') du réceptacle d'aiguille (8), avec le réceptacle d'aiguille (8).

5. Dispositif (1) selon la revendication 4, dans lequel le tiroir (13) présente un élément de séparation (15), avec lequel un capuchon de protection (16) peut être séparé d'une seringue (5) placée dans le tiroir (13).

6. Système de distribution d'un fluide dans des conditions aseptiques, comprenant un dispositif (1) selon l'une quelconque des revendications 1 à 8 et une seringue (5) pouvant être accouplée avec l'élément d'accouplement (4).

7. Système selon la revendication 6, dans lequel la seringue (5) est conçue sans tige de piston.

8. Système selon l'une quelconque des revendications 6 ou 7, dans lequel la seringue (6) présente une aiguille creuse ou canule (6) en particulier fixée à demeure sur le corps de seringue.

9. Système selon la revendication 8, dans lequel le dispositif (1) comprend un support de seringue (13), qui est conçu de sorte que l'aiguille de seringue ou canule (6) d'une seringue (5) placée dans celui-ci soit protégée.
